# EUROPEAN PATENT APPLICATION

(11) **EP 1 844 666 A1**
(43) Date of publication of application: **17.10.2007**
(21) Application number: 06380239.1
(22) Date of filing: 31.08.2006
(51) Int. Cl.: A23L 1/30, A23L 1/212

(54) **Olive pulp biomass with high phenolic antioxidant content, process for obtaining it and formulations and uses thereof**

(30) Priority: 02.09.2005 ES 200502143
(71) Applicant: Antas Pharma, S.A., 04638 Mojacar, Almeria (ES)
(72) Inventor: Esteban Morales, Manuel, 04638 Mojacar Almeria (ES)
(74) Representative: Carpintero Lopez, Francisco

(57) **Abstract**

The present invention provides an olive pulp biomass with a high content in phenolic antioxidants such as tyrosol, p-hydroxybenzoic acid, p-hydroxyphenylacetic acid, p-hydroxyphenylpropionic acid, vanillic acid, hydroxytyrosol, protocatechuic acid, 3,4-dihydroxyphenylglycol, beta-phenyllactic acid, p-hydroxycinnamic acid, cis-caffeic acid or trans-caffeic acid, in given concentrations. Said biomass is obtained by means of a process comprising adding a mixture of vitamin E and ascorbyl palmitate to the crushed olive paste prior to its centrifuging in order to separate the oil from the biomass. Likewise, the invention also provides formulations comprising said biomass and the use thereof in pharmaceutical, food or cosmetic applications.

## Description

### FIELD OF THE INVENTION

The present invention belongs to the field of plant biomasses, and more specifically to biomasses obtained from olive pulp. The invention refers. specifically to an olive pulp biomass with natural antioxidant properties, a process for obtaining it, formulations comprising said biomass and the use of these formulations in pharmaceutical, food or cosmetic applications.

### BACKGROUND OF THE INVENTION

The olive fruit is well known in the Mediterranean basin, it being one of the essential ingredients of its diet, both for cooking and raw. The composition of olives is complex, rich in oligoelements and other nutrients and with very healthy properties. Therefore, it is of interest to maintain the components of both the olive and its derivatives unaltered and stabilized for their subsequent use in food, pharmaceutics and cosmetics. The present invention discloses an olive pulp biomass that is homogenous and stable, that keeps the properties of its natural antioxidants and that is completely natural.

There are different types of polyphenols according to the type of olives. These may contain mainly polar compounds such as the glycosides oleuropein and ligstroside, which are precursors of the aglycone derivatives, compounds that are even more polar. Oleuropein aglycone is the ester of elenolic acid with 3,4-o-dihydroxyphenylethanol (hydroxytyrosol) and ligstroside aglycone is the ester of elenolic acid with 4-hydroxyphenylethanol (tyrosol), and these are formed when a glucose molecule of the corresponding glycoside is lost in the olive ripening process. Aglycones and their different derivatives are the most abundant phenols in ripe olives and in oil. The derivatives differ in their chemical structures, the ring being more open or closed, polyphenols tyrosol and hydroxytyrosol being the end products of the hydrolysis of aglycone derivatives of oleuropein and ligstroside.

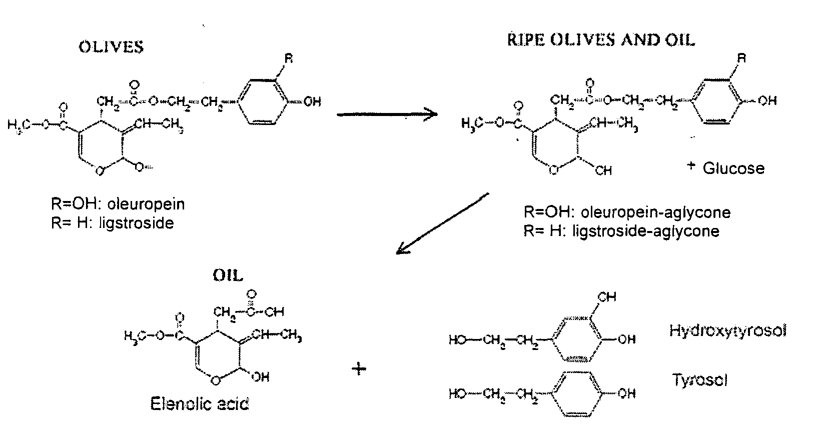

These are the most important polyalcohols, although other products may also be stressed, such as those described in Table I.

**Table I. Composition of the unsaponificable fraction of olives.**

| **Compound** | **Concentration** |
|---|---|
| Total hydrocarbons squalene | 300-700 mg/100 g |
| | 300-700 mg/100 g |
| Pigments chlorophylls | 0-9.7 mg/kg |
| Carotenoids | 0.50-10 mg/kg (in β-carotene) |
| lutein | 30-60% |
| β-carotene | 5-15% |
| Tocopherols | 70-300 mg/kg |
| α-tocopherol | > 93% |
| β and γ-tocopherol | < 10% |
| γ-tocopherol | < 1.5% |
| Sterols | 80-240 mg/100 g |
| β-sitosterol | 75-95 % |
| campesterol | 2-4% |
| stigmasterol | 1-2% |
| Δ 5-avenasterol | 3-14% |
| Δ 7-avenasterol | < 0.7% |
| Phenolic compounds | 50-800 mg/kg (in caffeic acid) |
| Triterpenic alcohols | 100-300 mg/100 mg |
| Aromatic compounds | |
| Alcohols | |
| Ketones | |
| Esters | |
| Ethers furanic derivatives | |

The composition in sterols, vitamin E and lutein, which are strong antioxidants, is to be stressed.

Phenol concentration in olives varies from 50 to 800 mg/kg with an average value of 180 mg/kg. Phenol concentration varies according to climate, olive type, farming area and fruit ripeness. Phenols in general, and ortho-dihydroxyphenol in particular, greatly contribute to broth stability.

The pharmacological interest of these products is mainly focused on the fact that they present important antioxidant properties, mainly ortho-dihydroxyphenolics, such as oleuropein and hydroxytyrosol, since they are great *in vitro* inhibitors of LDL oxidation (Visioli et al. Lipid 1999, suppl. S315). The antioxidant activity of hydroxytyrosol is due to both a metal ion chelator effect and a free radical sequestrant effect (Violi et al. Nutr Rev 1998; 142-47).

Oleuropein and hydroxytyrosol have demonstrated their activity as free radical sequestrants with a power similar to that of vitamin E, vitamin C and butylhydroxytoluene (Violi et al. Nutr Rev 1998; 142-47).

Other antioxidants present in olive pulp, such as lignans, have demonstrated their antioxidant activity *in vitro* (Owen et al. Food Chem Toxicol. 2000; 38.647-59), exhibiting the highest antioxidant capacity, followed by hydroxytyrosol and tyrosol (Owen et al. Euro. J. Cancer; 2000; 36.1235-47).

These compounds are difficult to extract from the olive pulp, remaining as such in the pulp biomass.

It has currently been demonstrated that UV rays induce serious injuries on the skin by generating highly reactive chemical species and reducing endogenous antioxidants. These studies demonstrate that hydroxytyrosol and its methylated derivatives have great antioxidant capacity, producing a differentiated cellular behavior of human melanoma cells by protecting them from radiation, thus its interest in functional foods and cosmetics (D'Angelo et al. Free Radic. Biol. Med 2005, April 1: 38(7):908-19).

Legar et al. (Eur J Clin Nutr. 2005 May; 59(5):727-30) have demonstrated a correlation between polyphenols and antithrombotic activity and likewise with platelet anti-aggregating activity, thus favoring blood circulation.

This antioxidant activity is present in the different parts of olives including the pulp, such as demonstrated in the work of Morillo et al. (J Agric Food Chem. 2005 Mar 23; 53(6):2002-8).

This antioxidant activity has been disclosed in the patent US 6,746,706 for its use as food additives. Indeed, the hydrophilic phase from pulp extraction is used as a whole in sauce preparations and in the food industry.

Likewise, it has recently been demonstrated that the processes involved in producing the pulps can influence olive antioxidant concentrations. Thus, Romero et al. (J Agric Food Chem. 2004 Feb 11; 52(3):479-84) have demonstrated that the phenol compositions in each phase are very different since hydroxytyrosol and tyrosol are present in the olive juice because they are more hydrophilic, whereas tyrosol and hydroxytyrosol acetates and lignans (1-acetoxypinoresin and pinoresinol) remain in the solid residue, being very sensitive to the oxidations inherent to the process.

Surprisingly, it has been discovered that adding natural antioxidants such as vitamin E and ascorbyl palmitate during the pulp-obtaining process prevents said unwanted oxidations and allows obtaining an olive pulp biomass with a high content in phenolic compounds with antioxidant properties.

### BRIEF DESCRIPTION OF THE DRAWINGS

Figure 1 shows the mass spectra for p-hydroxybenzoic acid (above) and the reference (below) from Wiley Library 275.
Figure 2 shows the mass spectra for p-hydroxyphenylacetic acid (above) and the reference (below) from Wiley Library 275.
Figure 3 shows the mass spectra for vanillol (above) and the reference (below) from Wiley Library 275.
Figure 4 shows the mass spectra for silanized hydroxytyrosol (above) and the reference (below) from Wiley Library 275.
Figure 5 shows the mass spectra for silanized p-coumaric acid (above) and the reference (below) from Wiley Library 275.
Figure 6 shows the mass spectra for silanized p-2-OH-glutaric acid (above) and the reference (below) from Wiley Library 275.
Figure 7 shows the mass spectra for silanized tyrosol (above) and the reference (below) from Wiley Library 275.
Figure 8 shows the mass spectra for syringaldehyde (above) and the reference (below) from Wiley Library 275.
Figure 9 shows the mass spectra for p-hydroxyphenylpropionic acid (above) and the reference (below) from Wiley Library 275.
Figure 10 shows the mass spectra for vanillic acid (above) and the reference (below) from Wiley Library 275.
Figure 11 shows the mass spectra for 3,4,5-trimethoxybenzoic acid (above) and the reference (below) from Wiley Library 275.
Figure 12 shows the mass spectra for protocatechuic acid (above) and the reference (below) from Wiley Library 275.
Figure 13 shows the mass spectra for syringic acid (above) and the reference (below) from Wiley Library 275.
Figure 14 shows the mass spectra for p-coumaric acid (above) and the reference (below) from Wiley Library 275.
Figure 15 shows the mass spectra for p-sinapic acid (above) and the reference (below) from Wiley Library 275.
Figure 16 shows the mass spectra for ferulic acid (above) and the reference (below) from Wiley Library 275.
Figure 17 shows the mass spectra for the esculetin derivative (above) and the reference (below) from Wiley Library 275.
Figure 18 shows the mass spectra for the caffeic acid derivative (above) and the reference (below) from Wiley Library 275.

### OBJECT OF THE INVENTION

The present invention, therefore, has as an object to provide an olive pulp biomass with high phenolic antioxidant content.

Another object of the invention is to provide a process for obtaining said biomass.

Likewise, another object of the invention is to provide a formulation comprising said biomass for pharmaceutical, food or cosmetic applications.

Finally, another object of the invention is to provide the use of said formulation for pharmaceutical, cosmetic or food applications.

### DETAILED DESCRIPTION OF THE INVENTION

The present invention provides an olive pulp biomass with high phenolic antioxidant content (hereinafter "biomass of the invention") comprising the following compounds with a concentration equal to or less than that indicated:

| Phenolic compound | Concentration (mg/kg) |
|---|---|
| Tyrosol | 172 |
| p-OH-benzoic acid | 27 |
| p-OH-Ph-acetic acid | 18 |
| p-OH-Ph-propionic acid | 32 |
| Vanillic acid | 34 |
| OH-tyrosol | 408 |
| Protocatechuic acid | 93 |
| 3,4-di-OH-Ph-glycol | 39 |
| beta-Ph-lactic acid | 12 |
| p-OH-cinnamic acid | 107 |
| Cis-caffeic acid | 47 |
| Trans-caffeic acid | 12 |
| Total phenolics | 1001 |

| | |
|---|---|
| p-OH-benzoic acid = p-hydroxybenzoic acid p-OH-Ph-acetic acid = p-hydroxyphenylacetic acid p-OH-Ph-propionic acid = p- hydroxyphenylpropionic acid OH-tyrosol = hydroxytyrosol 3,4-di-OH-Ph-glycol = 3,4-dihydroxyphenylglycol beta-Ph-lactic acid = beta-phenyllactic acid p-OH-cinnamic acid = p-hydroxycinnamic acid | |

In the context of the invention, the term "olive pulp biomass" refers to an organic natural material that is homogenous, doughy and viscous, originates from crushing olives, is rich in water, oils and natural active ingredients and which smells strongly of olive oil and has a characteristic bitter taste.

In a particular embodiment, the biomass of the invention comprises the following compounds with a concentration equal to that indicated:

| Phenolic compound | Concentration (mg/kg) |
|---|---|
| Tyrosol | 115 |
| p-OH-benzoic acid | 15 |
| p-OH-Ph-acetic acid | 11 |
| p-OH-Ph-propionic acid | 25 |
| Vanillic acid | 23 |
| OH-tyrosol | 260 |
| Protocatechuic acid | 80 |
| 3,4-di-OH-Ph-glycol | 20 |
| beta-Ph-lactic acid | 9 |
| p-OH-cinnamic acid | 95 |
| Cis-caffeic acid | 30 |
| Trans-caffeic acid | 5 |
| Total phenolics | 688 |

In another particular embodiment, the biomass of the invention comprises the following compounds with a concentration equal to that indicated:

| Phenolic compound | Concentration (mg/kg) |
|---|---|
| Tyrosol | 164 |
| p-OH-benzoic acid | 27 |
| p-OH-Ph-acetic acid | 18 |
| p-OH-Ph-propionic acid | 21 |
| Vanillic acid | 32 |
| OH-tyrosol | 397 |
| Protocatechuic acid | 91 |
| 3,4-di-OH-Ph-glycol | 14 |
| beta-Ph-lactic acid | 9 |
| p-OH-cinnamic acid | 107 |
| Cis-caffeic acid | 46 |
| Trans-caffeic acid | 9 |
| Total phenolics | 935 |

In another particular embodiment, the biomass of the invention comprises the following compounds with a concentration equal to that indicated:

| Phenolic compound | Concentration (mg/kg) |
|---|---|
| Tyrosol | 172 |
| p-OH-benzoic acid | 21 |
| p-OH-Ph-acetic acid | 14 |
| p-OH-Ph-propionic acid | 32 |
| Vanillic acid | 34 |
| OH-tyrosol | 408 |
| Protocatechuic acid | 93 |
| 3,4-di-OH-Ph-glycol | 39 |
| beta-Ph-lactic acid | 12 |
| p-Oh-cinnamic acid | 98 |
| Cis-caffeic acid | 47 |
| Trans-caffeic acid | 12 |
| Total phenolics | 982 |

In another aspect of the invention, a process is provided for obtaining the biomass of the invention comprising the following steps:
a) eliminating leaves and other residues from the olives to be treated;
b) washing the olives;
c) crushing the washed olives in order to form a homogenous paste;
d) adding vitamin E and ascorbyl palmitate to said paste; and
e) centrifuging the mixture obtained in d) in order to separate the oil from the olive pulp biomass with a high content in phenolic antioxidants.

In a particular embodiment of said process, a mixture of vitamin E and ascorbyl palmitate in an amount of 1 g/kg of biomass is added in step d) at room temperature and under an inert atmosphere.

Said process is a simple and industrial process performed at room temperature and under N₂ atmosphere in stainless steel reactors.

More specifically, the leaves and other residues such as rock, sand, etc., that accompany the olives when these are harvested are removed by means of a separator. Subsequently, the olives are washed with tap water at room temperature in the same separator. The washed olives are then added to a tight-closing metal mill (a hammer or ball mill, for example) loaded with N₂, since it is better at keeping antioxidant and oil stability, until achieving a homogenous paste. Said paste is subsequently arranged in a dough mixer, where 1 g/kg of vitamin E and ascorbyl palmitate is added to it, giving the mixture greater antioxidating power. This mixing process is performed for 20 minutes and under a nitrogen atmosphere in order to preserve the antioxidating properties of the already stabilized biomass. Afterwards, if necessary, the dough is heated to 20°C thus increasing its fluidity. Said dough is added to a two-phase centrifuge separator in which it is centrifuged at 5000 rpm, for example, extracting the oil and retaining a stabilized oily/hydrophilic dough that is rich in fibers, organic matter, micronutrients and oligoelements, and with the antioxidants of interest.

Finally, the biomass thus obtained is dosed and packaged under nitrogen atmosphere in 1 Kg doses and, finally, pasteurized.

In another aspect of the invention a formulation is provided for pharmaceutical, food or cosmetic applications comprising the biomass of the invention.

In a particular embodiment, said formulation also comprises one or more liposoluble vitamins: vitamins A, E, D and K, alone or combined.

In another aspect of the invention the use of a formulation as previously described is provided for pharmaceutical, cosmetic or food applications.

In the case of pharmaceutical applications, the formulation is used as tablets, capsules, pills, coated tablets, elixirs, solutions, suspensions, emulsions, syrups, granules, sachets, drinkable vials, ointments, creams, gels, salves, balsams or transdermal patches, for example. In order to do this the biomass of the invention is combined with suitable excipients and/or carriers.

In the case of cosmetic applications, the formulation is used as bath gels, shampoos, conditioners, face packs, soaps, deodorants, shaving creams, lotions, moisturizing cosmetic creams, nutritive cosmetic creams, cleansing creams, facial toners, body milks, sun creams, talc powders, makeups or lipsticks, for example. In order to do this the biomass is formulated with suitable excipients and with squalene, facilitating polyphenol absorption in the skin. Particularly, this pasteurized biomass is of great interest for its use in spa resorts for treating skin diseases and relaxing.

Likewise, in the case of food applications, the formulation is used as ready-made dishes, dietetic products, cereals, diary products, bakery and pastry products, gelatins, drinks, soft drinks, ice-creams, butter, soups, broths, juices, salad dressings or sauces, for example. In order to do this, the biomass is formulated with suitable ingredients according to the type of product to be prepared.

The following examples illustrate the invention and must not be considered as limiting the scope thereof.

### EXAMPLES

### Biomass production

100 Kg of harvested olives were weighed and added to a Pieralisi®-type olive washer, separating the leaves, stones and other foreign objects. The washed and undried olives were added to a metal mill and ground for 10 minutes until obtaining a homogenous dough under an inert atmosphere. The dough was then added to a mixer for 20 minutes under an inert atmosphere and at 20°C in order to obtain a homogenous mass. It was subsequently centrifuged in a Westfalia®-type two-phase centrifuge at 5000 rpm, separating the oil from the biomass. A moist and oily polyphenol-rich biomass weighing approximately 75 Kg was thus recovered.

Said biomass was pasteurized at 60°C for 30 minutes and then cooled quickly to 3°C.

Finally, a cosmetic cream was formulated at 10% (w/w) with a 1/1000 squalene content.

The characteristics of the average biomass obtained are described in Table II.

**Table II. Chemical Properties of the Biomass.**

| **Physico-Chemical Parameters** | **Value** |
|---|---|
| pH | 6.8 |
| Electric conductivity EC (mS/cm) | 1.2 |
| Organic Matter (%) | 60.3 |
| Organic Carbon (%) | 35.5 |
| Organic Nitrogen (%) | 1.1 |
| C/N | 32.2 |
| P (%) | 0.03 |
| Ca (%) | 1.2 |
| Mg (%) | 0.96 |
| Na (ppm) | 180.0 |
| K (%) | 0.29 |
| Fe (g/kg) | 2.6 |
| Cu (ppm) | 13.3 |
| Zn (ppm) | 9.8 |

### PHENOLIC DERIVATIVE IDENTIFICATION

### Analytic method used in identifying phenolic compounds in the obtained biomass.

### Sample preparation

100 g of a sample which came from three biomasses originating from three olive varieties (alberquina, picual and hojiblanca) that were subsequently acidified to pH 3.0 with HCl (1 N) and saturated with NaCl (Panreac) were used as a starting material, then proceeding to the extraction of the study compounds with 50 ml of diethyl-ether (HPLC-grade, Lab-Scan). The extraction was performed in a sequenced and continuous manner and the organic extracts obtained were jointly collected in a flask (in which they were desiccated with enough amount of anhydrous sodium sulfate (Panreac), and the solvent was then eliminated in a rotary evaporator (Buchi-R) at 30°C. Once the dry residue was obtained it was collected in a vial in 10 ml of methanol (HPLC-grade, Lab-Scan), it was lyophilized and the dry extract was then dissolved again in 1 ml of pyridine (Panreac). Finally, the samples were analyzed by gas chromatography/mass spectrometry in the following amounts:
- 100 microliters of sample + 50 microliters of silanizing agent
- **Silanizing agent:** N,O-bis-trimethylsilyltrifluoroacetamide (BSTFA) (Fluka)

5 microliters of the prepared sample were analyzed.

### Equipment description

- HEWLETT-PACKARD HP 6890 Series GS system gas chromatographer.
- HP-5MS (30 m - 0.25 mm) column, crosslinked 5% PH ME, Siloxane.
- Split/Splitless automatic capillary injector coupled to the system.
- HEWLETT PACKARD HP 5973 mass selective detector mass spectrometer.
- Gold quadrupole. Mass interval 0-800 amu.
- HEWLETT PACKARD Vectra, Pentium 5/150 computer system.

### Working conditions

The GC/MS results were obtained according to the following working conditions:
- GC Column. 1 ml/ml flow. Helium carrier gas and 5 microliter injection volume.
- Chromatographer oven at starting temperature of 80°C.
- Injector at 250°C.
- Temperature gradient: Starting at 80°C. Increasing 5°C/min up to
- 220°C. Increasing 10°C/min up to 310°C.
- Final chromatography time 47 min.
- Daily calibration.
- 0-70 eV ionizing source
- Mass range of 0-400 amu.
- Result treatment: Wiley spectra library 275.

### Commercial standards

Before analyzing the tested biomasses a phenolic derivative study was performed with Sigma commercial standards.

| **Phenolic compound** | **Retention time (min)** |
|---|---|
| p-hydroxybenzaldehyde | 10.93 |
| Tyrosol | 15.64 |
| 3-OH-Ph-acetic acid | 16.52 |
| p-OH-benzoic acid | 16.92 |
| p-OH-Ph-acetic acid | 17.20 |
| Vanillol | 17.25 |
| Syringaldehyde | 18.55 |
| p-OH-Ph-propionic acid | 19.77 |
| Vanillic acid | 19.92 |
| 3,4,5-trimethoxybenzoic acid | 21.15 |
| Protocatechuic acid | 21.16 |
| Syringic acid | 22.73 |
| p-coumaric acid | 23.36 |
| Sinapic acid | 25.80 |
| Ferulic acid | 26.30 |
| Esculetin | 26.35 |
| Caffeic acid | 24.38 |

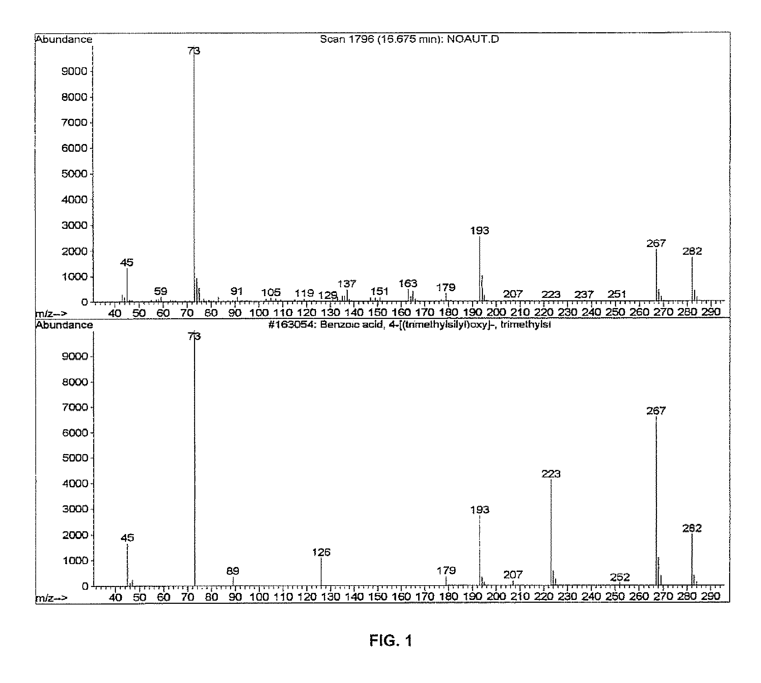

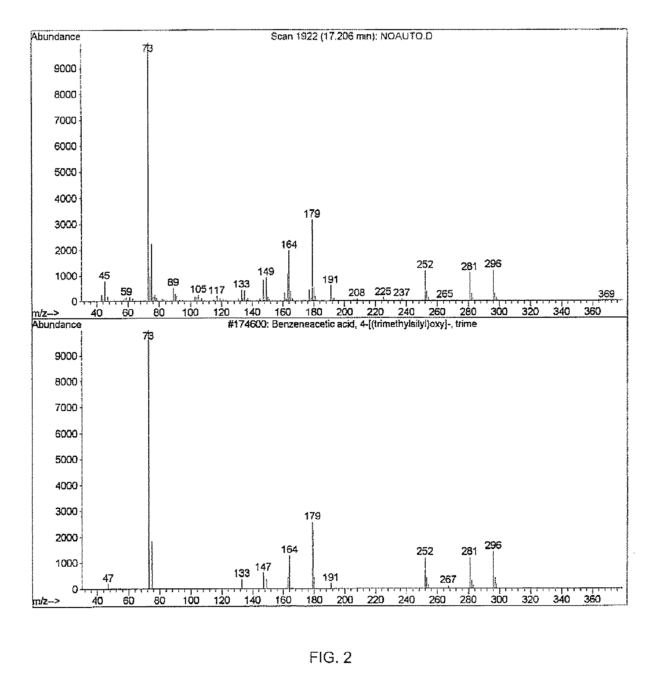

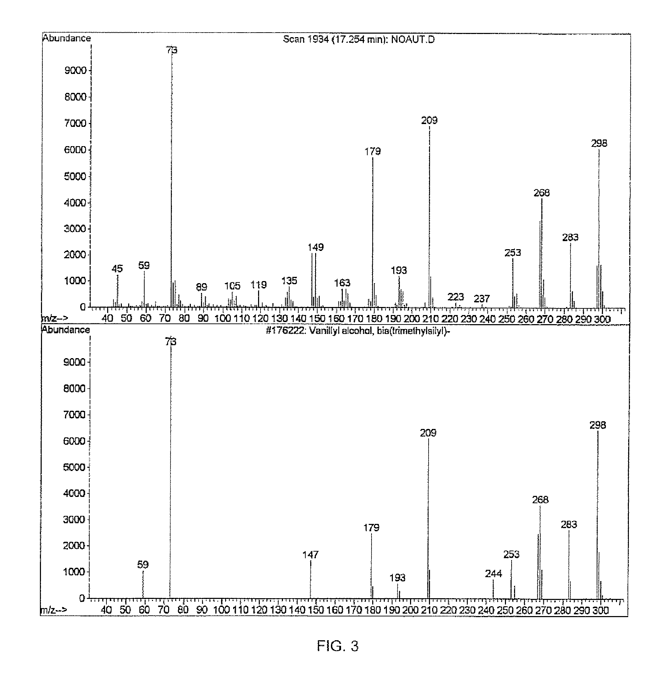

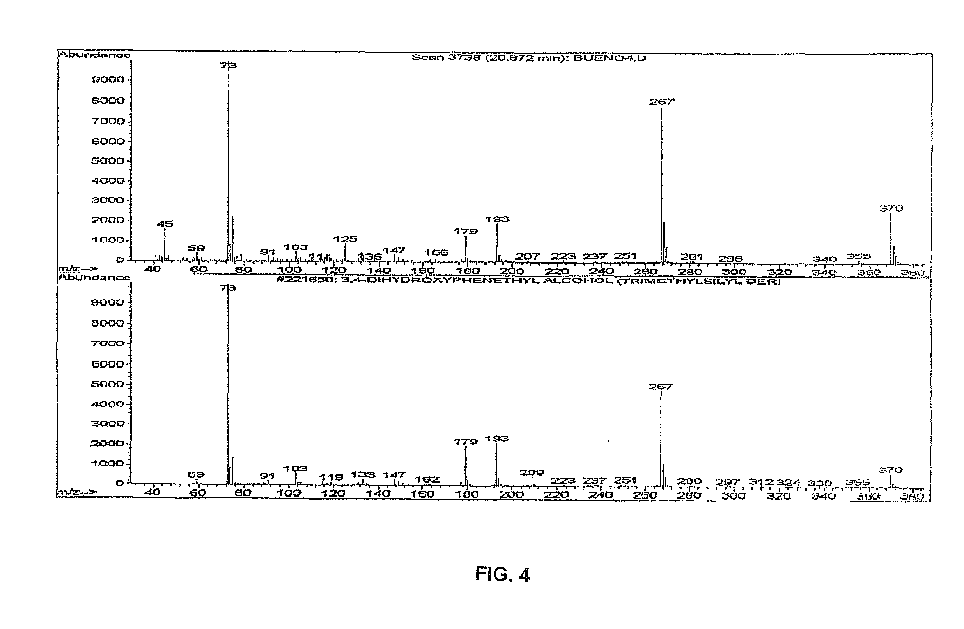

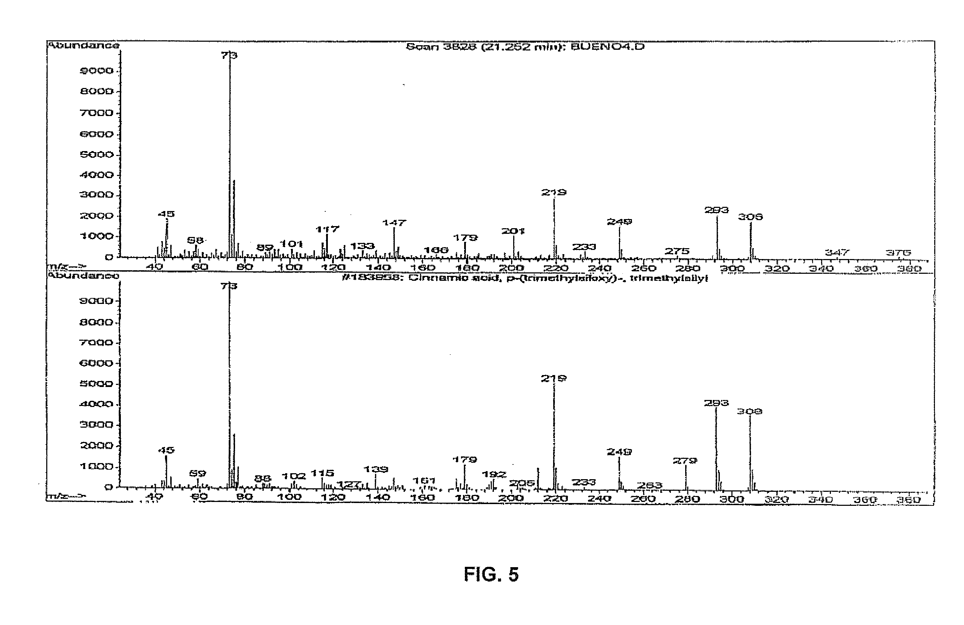

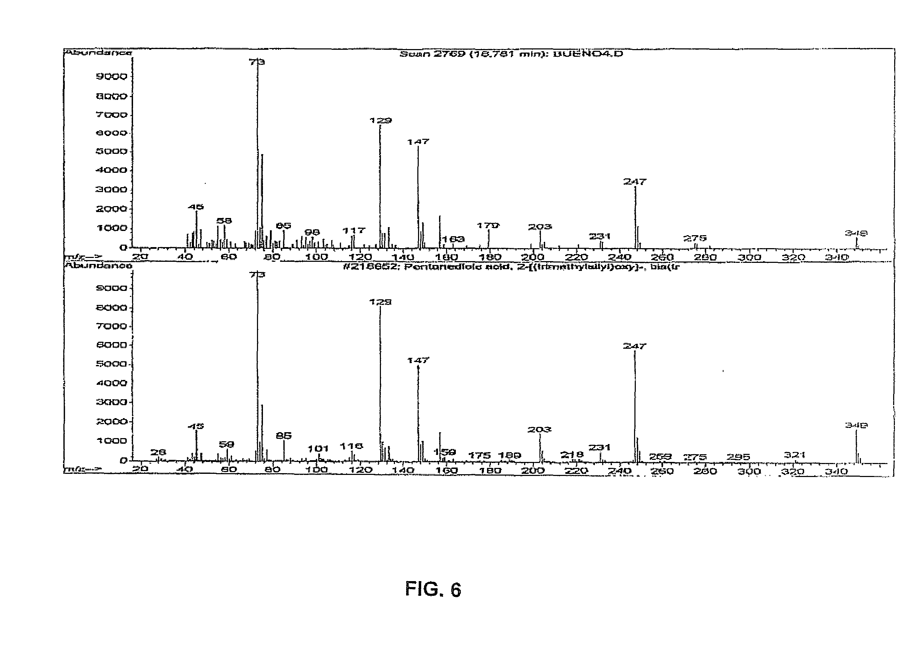

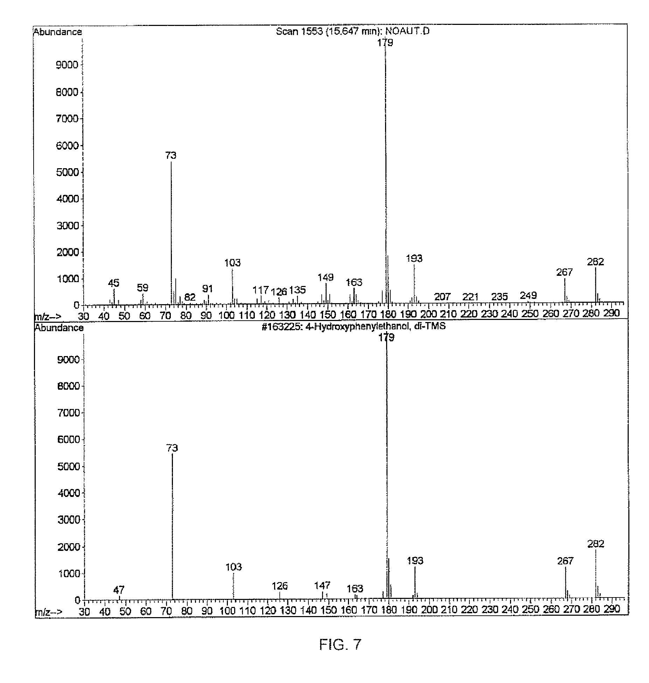

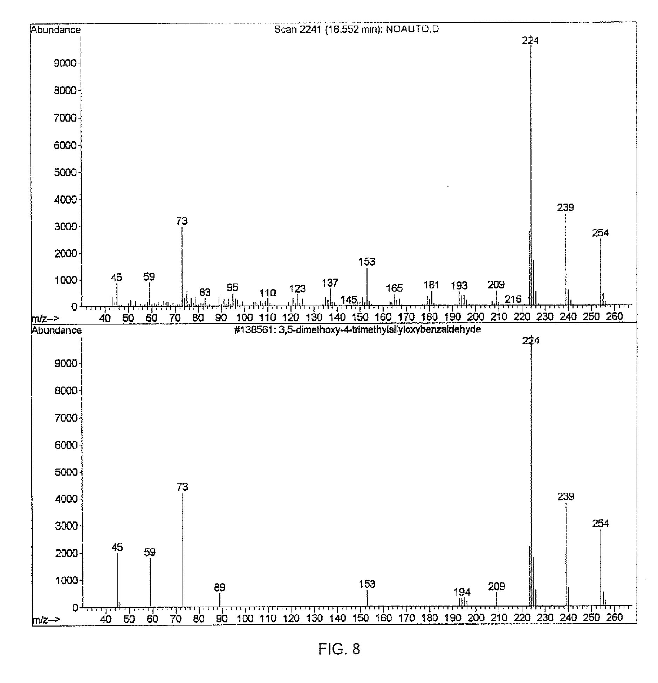

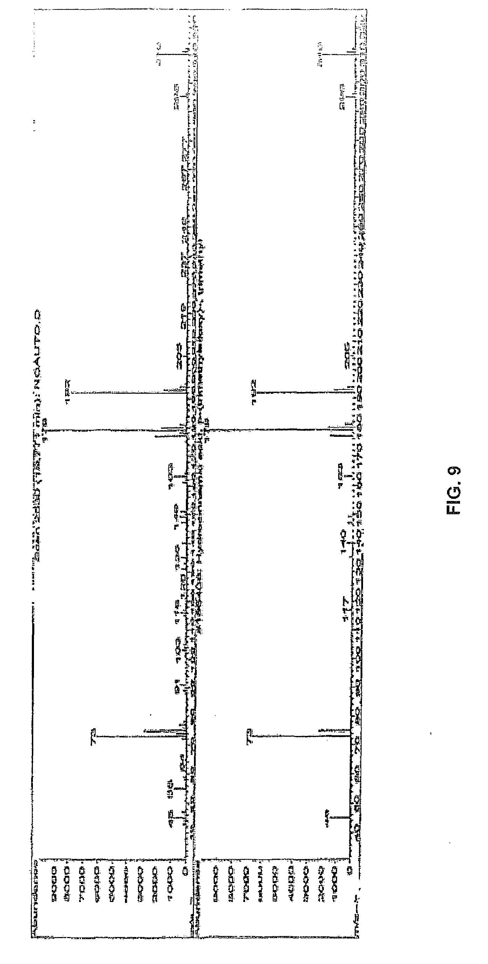

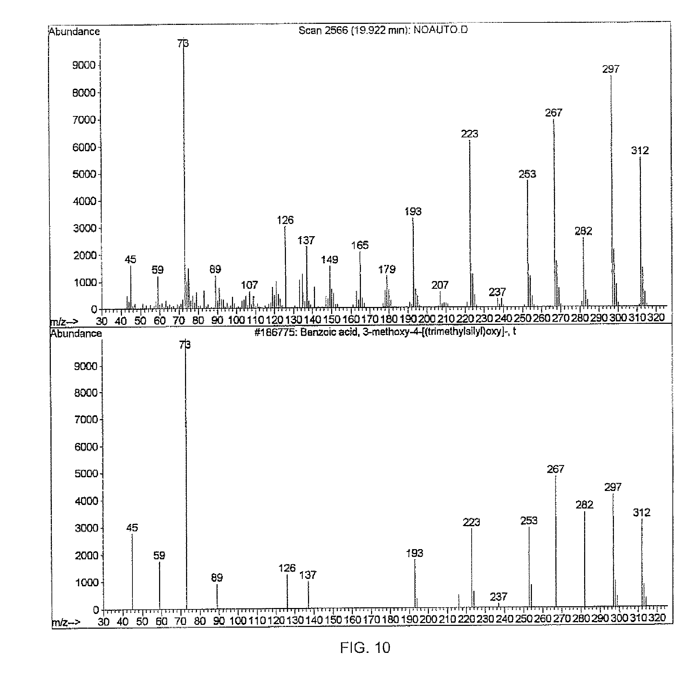

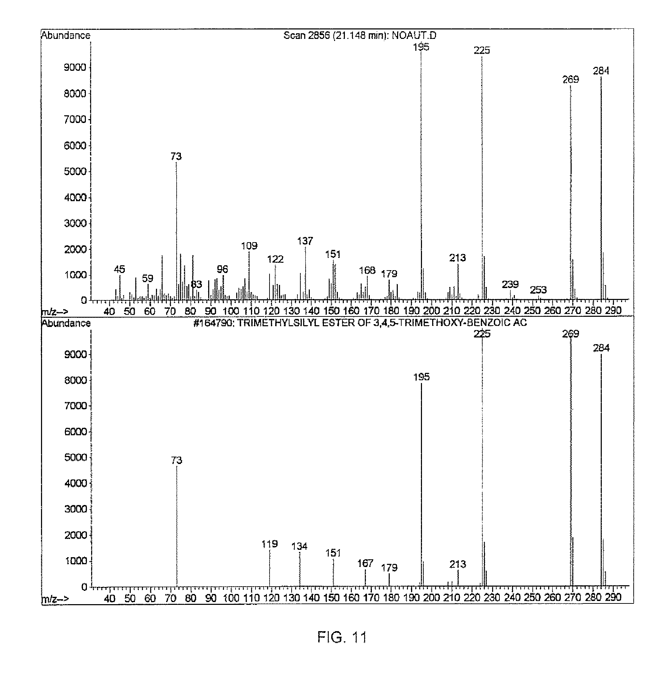

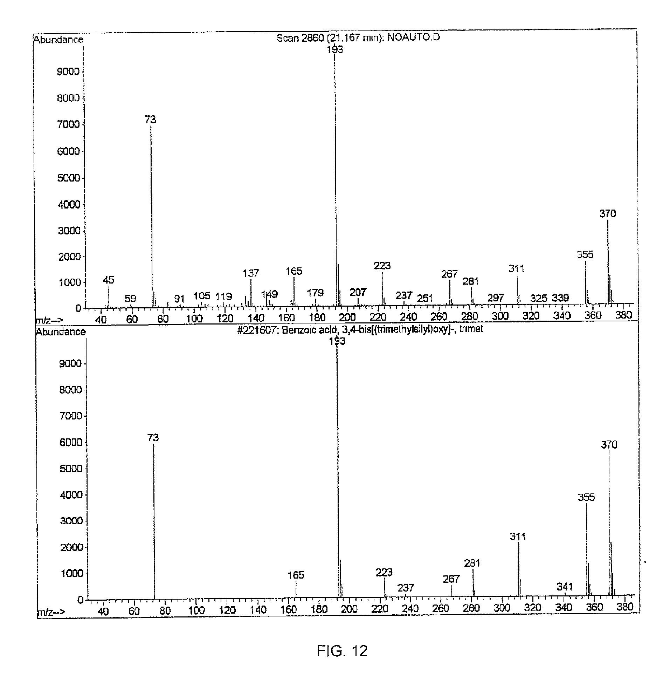

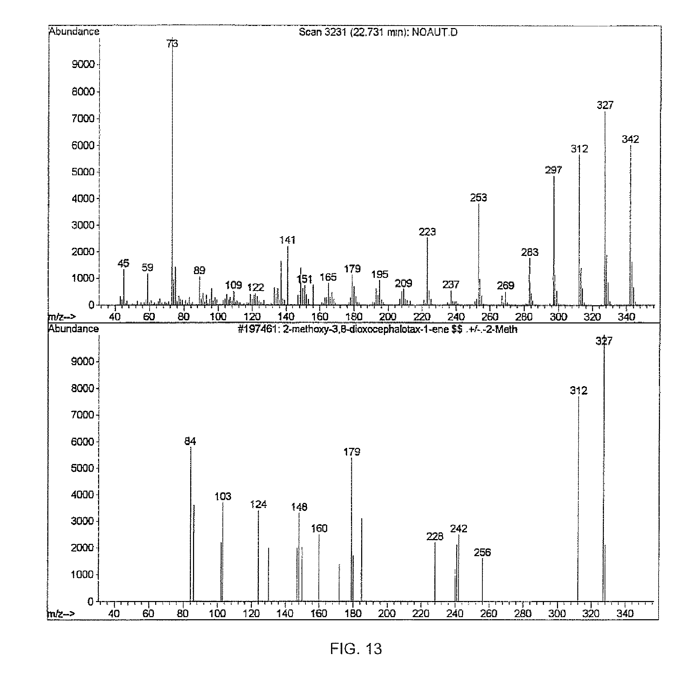

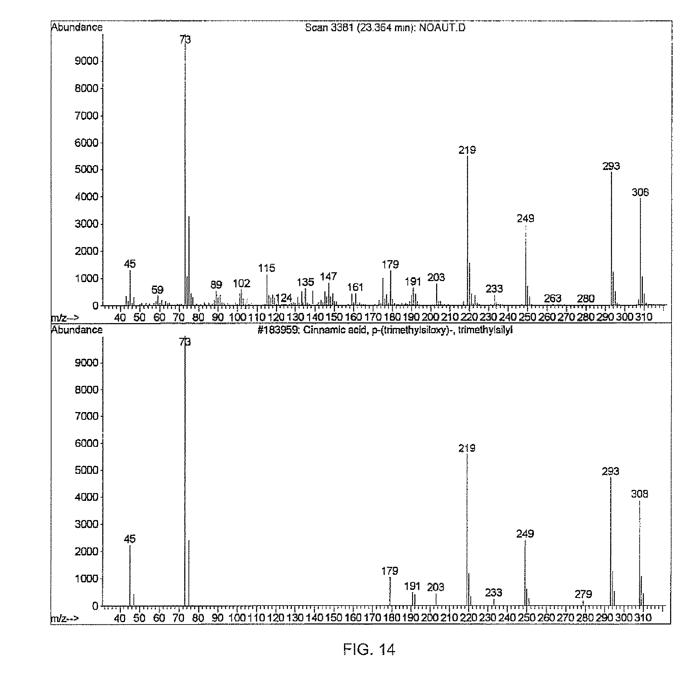

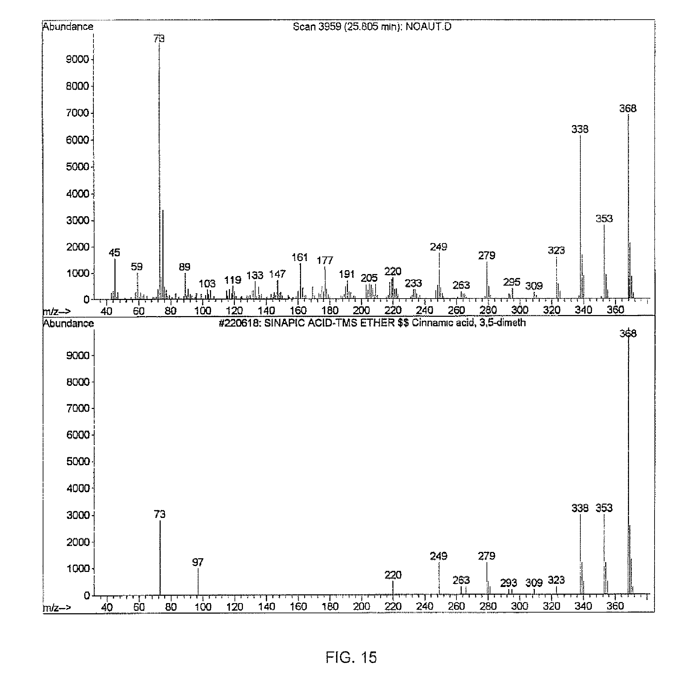

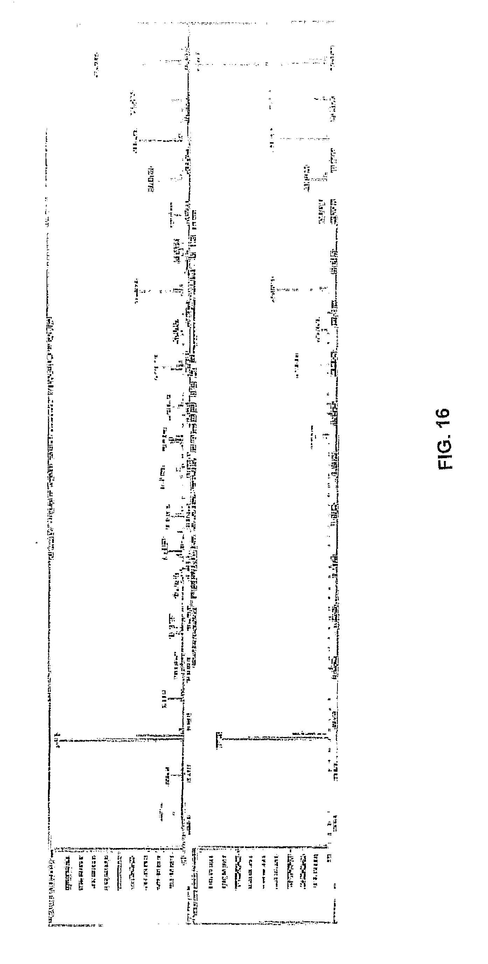

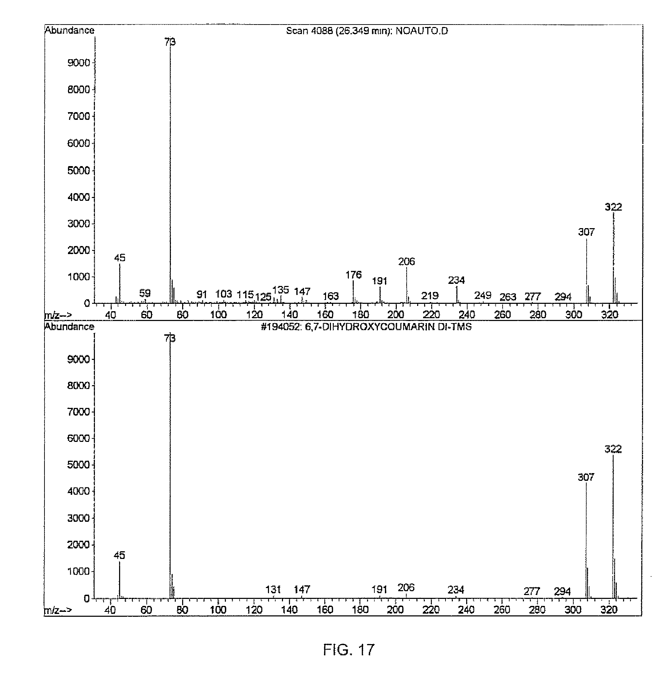

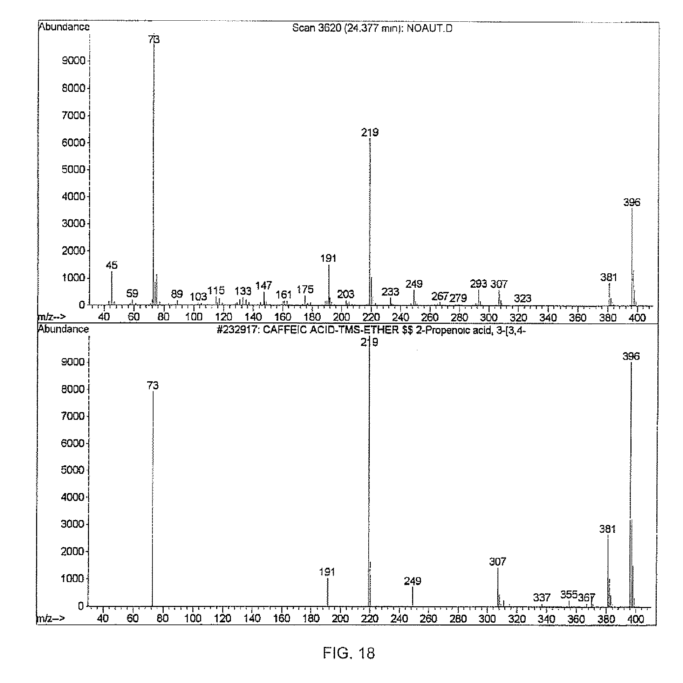

Figures 1-18 show the mass spectra of these commercial standards.

### Results

The following phenolic compounds were identified in the biomasses originating from olive varieties alberquina, picual and hojiblanca:
- Tyrosol
- beta-Ph-lactic acid
- p-OH-benzoic acid
- p-OH-Ph-acetic acid
- Vanillol
- p-OH-Ph-propionic acid
- Vanillic acid
- OH-tyrosol
- p-coumaric acid
- Protocatechuic acid
- 3,4-di-OH-Ph-glycol
- p-OH-cinnamic acid
- Cis-caffeic acid
- 3,4-dimethoxybenzoic acid
- Trans-caffeic acid

### PHENOLIC DERIVATIVE QUANTIFICATION

### Total phenolic derivative content

The total phenolic content of the biomasses for the varieties under study were the following:

| | |
|---|---|
| Alberquina: | 3.51 g/l |
| Hojiblanca: | 6.39 g/l |
| Picual: | 7.25 g/l |

The methodology followed for quantification of the different phenolic compounds of the biomasses was the internal standard technique, referring to an external calibration curve thereof. The internal Standard used in the biomass study should not be present in the samples, for which reason 18 biomasses were previously chromatographed, identifying the phenolic content. It was verified that 6,7-dihydroxycoumaric acid (esculetin) was not present in any samples, for which reason this compound was used as an internal reference for the quantitative study.

The preparation technique for the samples was that previously described, with the difference of the addition of the internal standard before extracting the sample. Losses on extraction are thus rejected since they are similar both for the internal standard and for the phenolic compounds under study.

### Results

| **Phenolic derivative** | **Alberquina** | **Hojiblanca** | **Picual** |
|---|---|---|---|
| Tyrosol | 115 | 164 | 172 |
| p-OH-benzoic acid | 15 | 27 | 21 |
| p-OH-Ph-acetic acid | 11 | 18 | 14 |
| p-OH-Ph-propionic acid | 25 | 21 | 32 |
| Vanillic acid | 23 | 32 | 34 |
| OH-tyrosol | 260 | 397 | 408 |
| Protocatechuic acid | 80 | 91 | 93 |
| 3,4-di-OH-Ph-glycol | 20 | 14 | 39 |
| beta-Ph-lactic acid | 9 | 9 | 12 |
| p-OH-cinnamic acid | 95 | 107 | 98 |
| Cis-caffeic acid | 30 | 46 | 47 |
| Trans-caffeic acid | 5 | 9 | 12 |

The concentration shown is the average of three determinations and is expressed in mg/kg.

## Claims

1. An olive pulp biomass with a high content in phenolic antioxidants **characterized in that** it comprises the following compounds with a concentration equal to or less than that indicated:
| Phenolic compound | Concentration (mg/kg) |
|---|---|
| Tyrosol | 172 |
| p-OH-benzoic acid | 27 |
| p-OH-Ph-acetic acid | 18 |
| p-OH-Ph-propionic acid | 32 |
| Vanillic acid | 34 |
| OH-tyrosol | 408 |
| Protocatechuic acid | 93 |
| 3,4-di-OH-Ph-glycol | 39 |
| beta-Ph-lactic acid | 12 |
| p-OH-cinnamic acid | 107 |
| Cis-caffeic acid | 47 |
| Trans-caffeic acid | 12 |
| Total phenolics | 1001 |

2. A biomass according to claim 1, **characterized in that** it comprises:
| Phenolic compound | Concentration (mg/kg) |
|---|---|
| Tyrosol | 115 |
| p-OH-benzoic acid | 15 |
| p-OH-Ph-acetic acid | 11 |
| p-OH-Ph-propionic acid | 25 |
| Vanillic acid | 23 |
| OH-tyrosol | 260 |
| Protocatechuic acid | 80 |
| 3,4-di-OH-Ph-glycol | 20 |
| beta-Ph-lactic acid | 9 |
| p-OH-cinnamic acid | 95 |
| Cis-caffeic acid | 30 |
| Trans-caffeic acid | 5 |
| Total phenolics | 688 |

3. A biomass according to claim 1, **characterized in that** it comprises:
| Phenolic compound | Concentration (mg/kg) |
|---|---|
| Tyrosol | 164 |
| p-OH-benzoic acid | 27 |
| p-OH-Ph-acetic acid | 18 |
| p-OH-Ph-propionic acid | 21 |
| Vanillic acid | 32 |
| OH-tyrosol | 397 |
| Protocatechuic acid | 91 |
| 3,4-di-OH-Ph-glycol | 14 |
| beta-Ph-lactic acid | 9 |
| p-OH-cinnamic acid | 107 |
| Cis-caffeic acid | 46 |
| Trans-caffeic acid | 9 |
| Total phenolics | 935 |

4. A biomass according to claim 1, **characterized in that** it comprises:
| Phenolic compound | Concentration (mg/kg) |
|---|---|
| Tyrosol | 172 |
| p-OH-benzoic acid | 21 |
| p-OH-Ph-acetic acid | 14 |
| p-OH-Ph-propionic acid | 32 |
| Vanillic acid | 34 |
| OH-tyrosol | 408 |
| Protocatechuic acid | 93 |
| 3,4-di-OH-Ph-glycol | 39 |
| beta-Ph-lactic acid | 12 |
| p-OH-cinnamic acid | 98 |
| Cis-caffeic acid | 47 |
| Trans-caffeic acid | 12 |
| Total phenolics | 982 |

5. A process for obtaining an olive pulp biomass with a high content in phenolic antioxidants according to any one of claims 1 to 4, **characterized in that** it comprises the following steps:
a) eliminating leaves and other residues from the olives to be treated;
b) washing the olives;
c) crushing the washed olives in order to form a homogenous paste;
d) adding vitamin E and ascorbyl palmitate to said paste; and
e) centrifuging the mixture obtained in d) in order to separate the oil from the olive pulp biomass with a high content in phenolic antioxidants.

6. A process according to claim 5, **characterized in that** in step d) a mixture of vitamin E and ascorbyl palmitate in an amount of 1 g/kg of biomass is added in step d) at room temperature and under an inert atmosphere.

7. A formulation for pharmaceutical, food or cosmetic applications **characterized in that** it comprises the olive pulp biomass with a high content in phenolic antioxidants according to any one of claims 1 to 4.

8. A formulation according to claim 7, **characterized in that** it comprises at least one liposoluble vitamin.

9. Use of a formulation according to any one of claims 7 to 8 for pharmaceutical applications, **characterized in that** the formulation is used as tablets, capsules, pills, coated tablets, elixirs, solutions, suspensions, emulsions, syrups, granules, sachets, drinkable vials, ointments, creams, gels, salves, balsams or transdermal patches.

10. Use of a formulation according to any one of claims 7 to 8 for cosmetic applications, **characterized in that** the formulation is used as bath gels, shampoos, conditioners, face packs, soaps, deodorants, shaving creams, lotions, moisturizing cosmetic creams, nutritive cosmetic creams, cleansing creams, facial toners, body milks, sun creams, talc powders, makeups or lipsticks.

11. Use of a formulation according to any one of claims 7 to 8 for food applications, **characterized in that** the formulation is used as ready-made dishes, dietetic products, cereals, diary products, bakery and pastry products, gelatins, drinks, soft drinks, ice-creams, butter, soups, broths, juices, salad dressings or sauces.
